# EUROPEAN PATENT APPLICATION

(11) **EP 3 549 539 A1**
(43) Date of publication of application: **09.10.2019**
(21) Application number: 17876462.7
(22) Date of filing: 24.11.2017
(51) Int. Cl.: A61B 17/56, A61F 2/28

(54) **PERSONALIZED ENDOPROSTHESIS DEVICE FOR SKELETAL BONES AND METHOD FOR THE IMPLANTATION OF SAME**

(30) Priority: 29.11.2016 RU 2016146763
(71) Applicant: Nikolaenko, Andrej Nikolaevich, Samara - Samarskaya Obl. 443052 (RU); Kolsanov, Aleksandr Vladimirovich, Samara - Samarskaya Obl. 443110 (RU); Popov, Nikolaj Vladimirovich, Samara - Samarskaya Obl. 443031 (RU)
(72) Inventor: Nikolaenko, Andrej Nikolaevich, Samara - Samarskaya Obl. 443052 (RU); Kolsanov, Aleksandr Vladimirovich, Samara - Samarskaya Obl. 443110 (RU); Popov, Nikolaj Vladimirovich, Samara - Samarskaya Obl. 443031 (RU)
(74) Representative: Jeck, Anton
(86) International application number: PCT/RU2017/000873
(87) International publication number: WO 2018/101858

(57) **Abstract**

A custom-made endoprosthetic device for skeletal bones comprising a body of a prosthesis duplicating the individual architecture of a patient's prosthetic bone and at least one fastening element, wherein said element is equipped with individual tendon fixation points constituting beds for passing surgical needles and bridges for the fixation of tendons with surgical sutures.

## Description

The invention relates to medicine, in particular to traumatology and orthopedics, neurosurgery, maxillofacial surgery, and stomatology and can be used for making custom-made endoprostheses of skeletal tubular and flat bones as well as facial skull and spinal bones when treating tumor and tumor-like patients and adult patients with bone injuries.

A method for individualized total joint replacement in patients with malignant joint tumors as per patent RF #2233639, 01/09/2003 priority date, published 08/10/2004, is known in the art; said method is applied as follows: the tumor is removed by segmental osteoarthrotomy or total joint resection, the resulting bone defect is filled with bone cement with antibiotics, which is molded by hand to the anatomical configuration and size of the removed bone segment or the entire joint; in the second stage the prepared and obtained total individualized endoprosthesis is implanted into the musculoskeletal bed maintained by the bone cement.

A known method for making hip joint components by digital techniques described in "Medical Technology" #3, 2016, pp 43-46 comprises CT-scanning, 3D scanning, and 3D printing, followed by making an endoprosthesis from titanium powder by selective laser sintering.

Said methods, however, don't succeed in attaining a precise volume of the removed segment of the affected bone and don't lend themselves to creating a custom-made endoprosthesis complete with muscle, tendon, and joint capsule fixations points.

The closest method to the claimed invention in the technical essence thereof is the method of creating individualized orthopedic implants according to patent application AU2015226831 (A1), published WO2015131234 (A1), priority 20140304. According to said patent, during a 3D scanning of the bones, a resected bone segment is virtually removed therefrom, a 3D digital model of the removed bone segment is obtained, and comparing the 3D image and the corresponding digital 3D images of the resected bone segment, the volume of the bone tissue that had been removed is assessed. An assessment of the volume of the removed bone segment is then used to make custom-made orthopedic implants that essentially correspond to the configuration of the volume of the removed bone tissue; such implant fits exactly to substantially repair the biomechanical bone function.

The disadvantage of said method is that it requires a two-stage surgical intervention: the oncological stage of removing the tumor and the orthopedic stage of endoprosthetics. Said tactics results in additional surgical trauma and the ensuing adverse effects. In addition, an endoprosthesis with individualized muscle, tendon, and joint capsule fixations points cannot be made according to said method.

The proposed invention is intended to achieve the following technical result: reduced recovery time of the anatomy of the removed bone segment and reduced periods of functional and social adaptation of patients due to determining the precise volume of the removed bone segment, which results in making an endoprosthesis with individualized muscle, tendon, and joint capsule fixations points.

The set objective is achieved with a custom-made endoprosthetic device for skeletal bones comprising a body of the prosthesis duplicating the individual architecture of a patient's prosthetic bone and at least one fastening element, wherein said element is equipped with individual tendon fixation points, said fixation points are beds for passing surgical needles and bridges for the fixation of tendons with surgical sutures. A method of implanting a custom-made endoprosthesis of skeletal bones is realized as follows: prior to surgical intervention, a virtual 3D template exactly duplicating the contours of the bone to be resected and facilitating a segmental resection of the affected part thereof as well as a custom-made endoprosthesis with individuaized muscle, tendon, and joint capsule fixations points marked thereupon are made based on contrast-enhanced CT scans; said template and the custom-made endoprosthesis with the desired characteristics are then reproduced in a material form, followed by a one-stage surgical intervention; after the surgical approach in the resection zone, the template is placed on the segment to be resected and filing is performed, wherein the segmental bone resection is performed strictly along the template and the contour of the endoprosthesis exactly matches the contour of the resected bone; the endoprosthesis is then implanted and the tendons are fixed to the individual points. The proposed implantation method of a custom-made endoprosthesis of skeletal bones is performed as a single surgical intervention: removal of the affected bone segment by resection along an individual template followed by custom-made endoprosthetics, which reduces postsurgical complications in a patient. The presence of individualized muscle, tendon, and joint capsule fixation points helps muscles return to their initial physiological place. Individualized muscle, tendon, and joint capsule fixation points constitute a bed for passing surgical needles and bridges for the fixation of tendons with surgical sutures. The monofilament surgical suture used for suturing tendons to the fixation point is dissolved within 30 days, during which time, the tendon tissue becomes integrated into the bioactive coating of the endoprosthesis and acquires a physiological position. The proposed method for custom-made endoprosthetics can be used for reconstruction of lost anatomical bone segments in major surgery of tumor and tumor-like patients and for trauma and for prosthetics of both skeletal tubular and flat bones, while resulting in a more precise calculation of the tissue volume to be removed intraoperatively and the best reconstruction of the removed bone segment by implantation of a custom-made endoprosthesis with individual muscle, tendon, and joint capsule fixation points, and, as a result, a fuller reconstruction of bone anatomy. A custom-made endoprosthesis with individual muscle, tendon, and joint capsule fixation points may be made, for example, by selective laser sintering of fine powdered titanium BT1-00, material specifications 1791-001-11805089-2014, the chemical composition of the powder is per GOST 19807-91. The custom-made endoprosthesis according to the aforementioned method has a custom-made anatomical contour and the curvature of joint surfaces. The segment resection is conducted strictly along the template in order to precisely adhere to the resection parameters. The contour of the endoprosthesis fully corresponds to the contour of the bone to be resected. For better integration, a bioactive material is coated onto the surface of the endoprosthesis in points of contact with soft tissue. The custom-made endoprosthesis of skeletal bones consist of a body of the prosthesis and at least one fastening element. The type and the number of fastening elements depends on the type of the prothesized bones, i.e. tubular or flat skeletal bones. The body of the prothesis fully replicates the individualized architecture of the patient's prothesized bone and is equipped with individualized muscle, tendon, and joint capsule fixations points of the patient to fully reconstruct the bone anatomy. The individualized tendon fixation points are beds for passing surgical needles and bridges for the fixation of tendons with surgical sutures. The custom-made endoprosthetic device for skeletal bones is exemplified by an endoprosthesis of the human anterior pelvic ring. The endoprosthesis of the human anterior pelvic ring, same as the pubic bone, consists of the superior ramus, the inferior ramus, and the body of the endoprosthesis. The endoprosthesis is fixed on the pelvis with fastening clamps, each of which corresponds to the patient's bone shape. In order to minimize the operating time, soft tissue trauma, and individualized search for the pelvic integrity reconstruction, the endoprosthesis with fastening clamps precisely follows the complex individual pelvic architecture. In the area of pubic symphysis, the fastening clamp closes the anterior pelvic ring by crossing over to the contralateral pubic bone. The endoprosthesis is contemplated to have individual muscle and tendon fixations points marked with special 3D-modeling programs. For better integration, the surface of the endoprosthesis is coated with a bioactive material in the points of contact with soft tissue. The custom-made endoprosthesis has a custom designed anatomical contour and the curvature of joint surfaces. Moreover, the segment resection is conducted strictly along the individualized template in order to precisely adhere to the resection parameters. The contour of the endoprosthesis exactly matches the contour of the resected bone. Fastening the endoprosthesis to the patient's pelvis with fastening clamps, which duplicate the individual anatomical contour of the patient's pelvis, reduces operating time and blood loss and moreover, it eliminates the need for intraoperational endoprosthesis fashioning.

The essence of the invention is illustrated graphically:
Fig. 1 Front view of a pelvic ring with an endoprosthesis
Fig. 2 Endoprosthesis of a segment of the pelvic ring
Fig. 3 Template for pelvic ring resection
Fig. 4 Pelvic ring with the template and filing planes

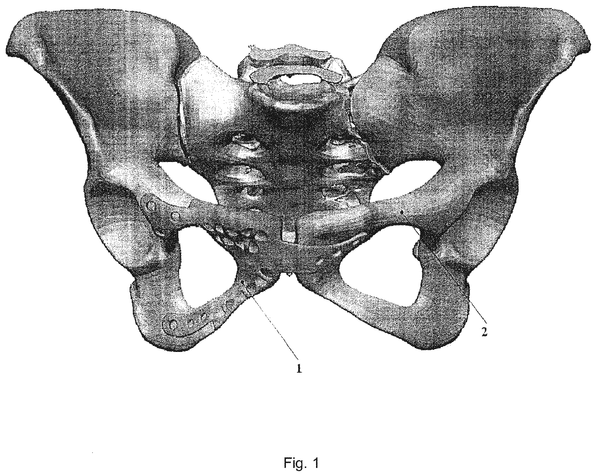

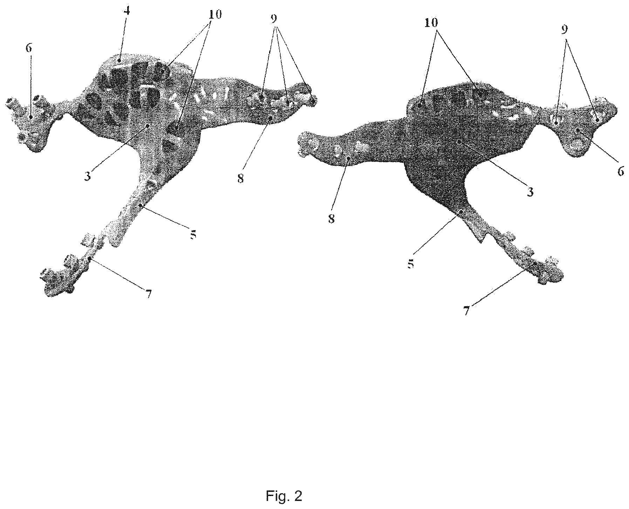

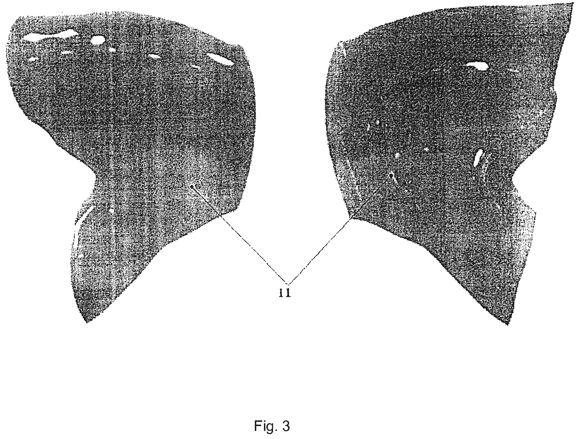

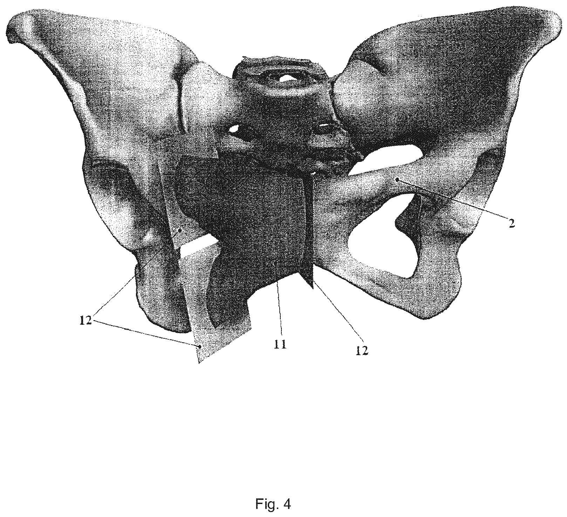

Fig. 1 shows 1 - endoprosthesis, 2 - pelvic ring. Fig. 2 shows 3 - body of endoprosthesis, 4 - superior ramus, 5 - inferior ramus, 6 - fastening clamp (ipsilateral pubic bond), 7 - fastening clamp (ischial bone), 8 - fastening clamp (contralateral pubic bone), 9 - fastening screws, 10 - fixation points. Fig. 3 shows 11 - template. Fig. 4 shows 2- pelvic ring, 11 - template, 12 - filing planes.

The proposed method for implanting a custom-made endoprosthesis of skeletal bones is realized as follows: prior to surgical intervention, virtual 3D template 11 exactly duplicating the contours of the bone to be segmentally resected and a custom-made endoprosthesis 1 model with individual muscle, tendon, and joint capsule fixations points 10 marked thereupon with a special computer program are made based on Omnipaque 50 ml contrast-enhanced CT scanning. The data is then converted into the STL format and then further processed in a 3D-printer software. Material copies of template 11 (Fig. 3) and endoprosthesis 1 (Fig. 2) are then prepared based on the data specified, for example, a stereolithographic print of template 11 for resection is made on a 3D-printer from temperature-resistant binary polyurethane and a stereolithographic print of endoprosthesis 1 is made on a 3D-printer, for example, from BT1-00 titanium powder. Following a surgical approach in the resection zone, template 11 is placed on the segment of the pelvic ring to be resected and filing is performed along filing planes 12 (Fig. 4) in three points with a saw. The thickness of the effective area of the saw blade (3 mm) has to be taken into consideration. The removed bone segment is sent for histological testing. The next stage is reconstruction, endoprosthesis 1 implantation. Endoprosthesis 1 of pelvic ring bones 2, as well as the patient's pubic bone of pelvic ring 2 consists of superior ramus 4, inferior ramus 5, and body of endoprosthesis 3. Endoprosthesis 1 is fastened to the patient's pelvic ring 2 with fastening clamps 6, 7, and 8, each of which corresponds to the patient's bone (Fig. 1). Fastening clamp 6 is for the ipsilateral pubic bone, i.e. on its own side; fastening clamp 7 for correlating with the ischial bone and fastening clamp 8 for the contralateral pubic bone are on the opposite side (Fig. 2). The clamps are fastened with screws 9 through 5.0 mm openings in clamps 6, 7, and 8, which are thin plates, 2 mm thick. Fastening clamps 6, 7, and 8 fully duplicate the complex individual architecture of the patient's pelvic ring 2. In the pubic symphysis, fastening clamp 8 closes the anterior pelvic ring by crossing over to the contralateral pubic bone. The endoprosthesis provides fixation points 10 for muscles and tendons that are fastened to a segment of patient's anterior pelvic ring 2. Individual muscle, tendon, and joint capsule fixations points 10 are beds for passing surgical needles and bridges for the fixation of tendons with surgical sutures. The monofilament surgical suture used for suturing tendons to the fixation point is dissolved within 30 days, during which time, the tendon tissue is integrated into the bioactive coating of endoprosthesis 1 and acquires a physiological position.

The combined features are novel and provide a one-stage surgical intervention for a bone resection and implementation of a custom-made endoprosthesis of the lost bone segment while reducing the period of functional and social adaptation of patients.

## Claims

1. A custom-made endoprosthetic device for skeletal bones comprising a body of a prosthesis duplicating the individual architecture of a patient's prosthetic bone and at least one fastening element, wherein said element is equipped with individual tendon fixation points constituting beds for passing surgical needles and bridges for the fixation of tendons with surgical sutures.

2. A method of implanting a custom-made endoprosthesis of skeletal bones, said method is conducted as follows: prior to surgical intervention, a virtual 3D template exactly duplicating the contours of the bone to be resected and facilitating a segmental resection of the affected part thereof as well as a custom-made endoprosthesis with individual muscle, tendon, and joint capsule fixations points marked thereupon are made based on contrast-enhanced CT scanning; said template and said custom-made endoprosthesis with the desired characteristics are then reproduced in a material form, followed by a one-stage surgical intervention; after the surgical approach in the resection zone, the template is placed on the segment to be resected and filing is performed, wherein the segmental bone resection is performed strictly along the template and the contour of the endoprosthesis exactly matches the contour of the resected bone; the endoprosthesis is then implanted and the tendons are fixed to the individual points
